# EUROPEAN PATENT APPLICATION

(11) **EP 3 812 494 A1**
(43) Date of publication of application: **28.04.2021**
(21) Application number: 19866549.9
(22) Date of filing: 27.09.2019
(51) Int. Cl.: D04H 1/425, D06M 16/00, A45D 44/00, A61K 8/02, A61F 13/00, A61L 15/36

(54) **BIO-CELLULOSE SHEET AND PREPARATION METHOD THEREOF**

(30) Priority: 27.09.2018 KR 20180115318; 26.09.2019 KR 20190119146
(71) Applicant: LG CHEM, LTD., Yeongdeungpo-gu, Seoul 07336 (KR)
(72) Inventor: KANG, Soonhee, Daejeon 34122 (KR); PARK, Minsung, Daejeon 34122 (KR)
(74) Representative: Hoffmann Eitle
(86) International application number: PCT/KR2019/012635
(87) International publication number: WO 2020/067798

(57) **Abstract**

The present disclosure provides a bio-cellulose sheet including a nonwoven fabric and bio-cellulose and having a structure in which the nonwoven fabric fiber and the bio-cellulose fiber are entangled, a preparation method thereof, and a beauty pack including the sheet.

## Description

### [TECHNICAL FIELD]

### CROSS CITATION TO RELATED APPLICATION(S)

This application claims the benefit of priority from Korean Patent Application No. 10-2018-0115318 filed on September 27, 2018 and Korean Patent Application No. 10-2019-0119146 filed on September 26, 2019 with the Korean Intellectual Property Office, the full disclosures of which are incorporated herein by reference.

The present disclosure relates to a bio-cellulose sheet including a nonwoven fabric and bio-cellulose and having a structure in which the nonwoven fabric fiber and the bio-cellulose fiber are entangled, a preparation method thereof, and a beauty pack or a medical patch including the sheet.

### [BACKGROUND ART]

Sheets used in the cosmetic and medical field refer to a cloth that can carry a cosmetic or pharmaceutical ingredient in the form of a liquid, cream, or emulsion and adhere it to the skin. Sine such a sheet can carry various effective ingredients, the effective ingredients penetrate into the skin upon adhesion to the skin to exhibit a cosmetic or therapeutic effect. Therefore, these sheets may be used as a beauty pack for moisturizing, whitening and nourishing the skin, or may be used as a medical patch for treatment of burns or wounds.

Sheets have been produced using a variety of materials. The initially developed sheets were made of nonwoven materials, and cosmetics with the purpose of providing whitening, anti-wrinkle effects, supplying nutrition or the like had been applied to nonwoven fabrics blended with rayon and polypropylene. Nonwoven fabrics are easy to secure in materials, inexpensive, and thus distributed in large quantities at low prices on the market. Subsequently, cotton materials for replacing nonwoven fabric have been developed, and pure cotton, which is a natural fiber, was used instead of synthetic blended rayon was used to minimize skin irritation and improve absorbency and adhesive strength. In addition, sheets made of hydrogel materials have been developed, which improve the disadvantages of the existing sheets made of nonwoven fabric or cotton materials through which essence flows down during use, and deliver nutrients or active ingredients deep into the skin, thereby accelerating the growth of beauty packs. However, the hydrogel sheets had a problem that causes skin troubles due to some synthetic cross-linking agents.

Thus, sheets that have recently gained much interest are bio-cellulose materials, which use natural bio-cellulose obtained by culturing microorganisms. A bio-cellulose sheet is a natural material, and thus has no side effects on the body, is environmentally friendly, has excellent adhesive strength and wearing feeling, and has a moisture content of 10 times or more compared to conventional nonwoven fabrics or cotton materials, whereby there is an advantage in that effective ingredients can be sufficiently provided to the skin and can penetrate deep into the skin.

Meanwhile, bio-cellulose has a problem that because of its softer and smoother texture compared to vegetable cellulose, it can be easily damaged (teared) and it is difficult to handle. Nevertheless, when a bio-cellulose is prepared thickly to reduce the damage of the bio-cellulose sheet, it takes a long time, and due to its large thickness, there is a limitation in sufficiently supplying effective nutrients to the skin, and in addition, the bent part of the skin may be not fully covered.

In this regard, in the case of currently commercialized bio-cellulose sheet, in order to prevent the sheets from tearing and facilitate handling of the sheets, it is provided in a state where a support film is laminated or attached to one side or both sides of the sheets to fix the shape after preparation of the bio-cellulose sheets. Examples of the support film include a nonwoven fabric film, a netlike fiber cloth film, a polymer thin-membrane film, a waterproof cloth film, a polymer-foam film, or a paper-like film, and the user must remove the film immediately before use.

However, when the support film is attached to the bio-cellulose sheet, an additional process of laminating the support film is required after preparing the bio-cellulose sheet, and the preparation cost may increase due to the raw cost of the support film. Further, it may be inconvenient for users to remove the support film every time when the product is used.

### [DETAILED DESCRIPTION OF THE INVENTION]

### [TECHNICAL PROBLEM]

Accordingly, there is a need to develop an improved bio-cellulose sheet which can overcome the weakness in terms of tearing and handling of the sheet, while maintaining excellent adhesive strength, wearing feeling, and absorbency, which are advantages of the bio-cellulose sheet.

### [TECHNICAL SOLUTION]

In one aspect, there is provided an improved bio-cellulose sheet which is resistant to tearing and is easy to handle even without using a support film, and maintains adhesive strength, wearing feeling, and absorbency, which are advantages of the bio-cellulose sheet.

In a specific embodiment, there is provided a bio-cellulose sheet including a nonwoven fabric and bio-cellulose and having a structure in which the nonwoven fabric fiber and the bio-cellulose fiber are entangled.

There is provided a method for producing the bio-cellulose sheet, including the steps of: impregnating a nonwoven fabric with a bio-cellulose culture solution; and culturing the nonwoven fabric impregnated with the culture solution.

There is provided a beauty pack including the bio-cellulose sheet.

There is provided a medical patch including the bio-cellulose sheet.

### [ADVANTAGEOUS EFFECTS]

The bio-cellulose sheet of the present disclosure has excellent adhesive strength, wearing feeling, and absorbency, is resistant to tearing and is easy to handle even when an additional support film is not laminated, and can be used immediately. In addition, the preparation process is facilitated and the amount of the microbial culture solution used is reduced, thereby being effective for reducing the preparation time and cost.

### [BRIEF DESCRIPTION OF THE DRAWINGS]

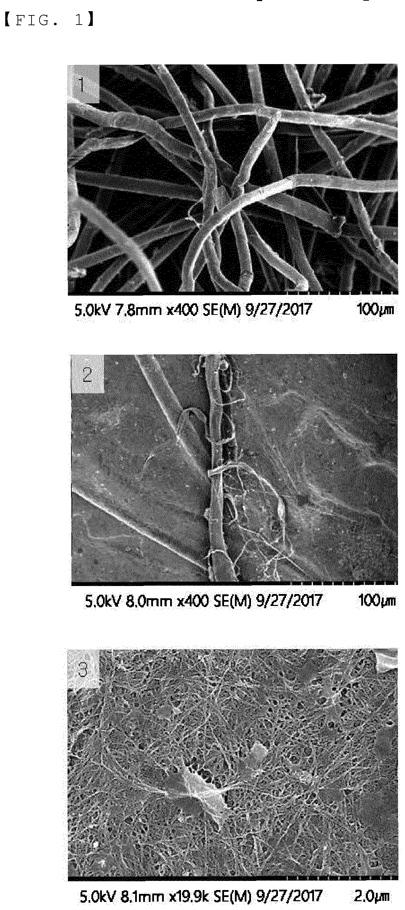

FIG. 1 shows scanning electron microscope (SEM) images of the bio-cellulose sheet according to one embodiment. Image No. 1 shows one surface of the bio-cellulose sheet on which bio-cellulose is not formed and only the nonwoven fibers exist. Images Nos. 2 and 3 show another surface of the bio-cellulose sheet in which the bio-cellulose fibers having nano-sized diameters and the microfibers of the nonwoven fabric are entangled.

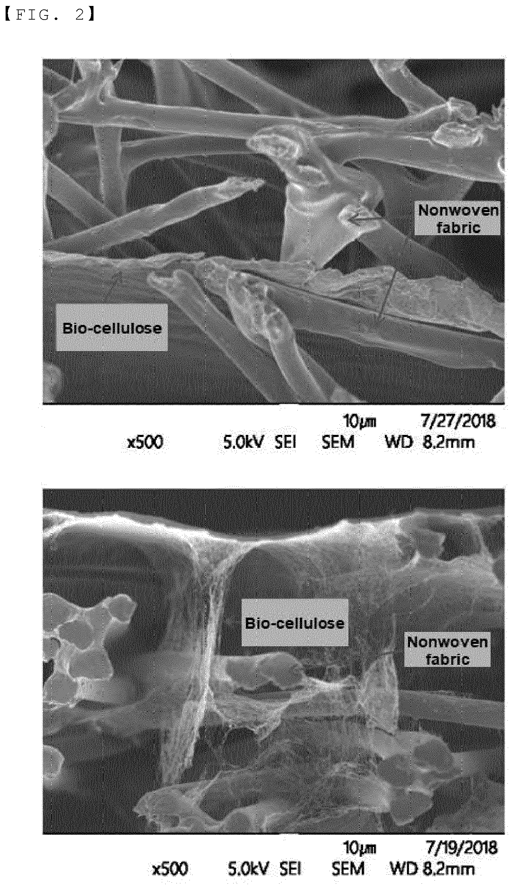

The upper image of FIG. 2 is a SEM image of the cross-section of a conventional bio-cellulose sheet in which nonwoven films are laminated on both sides, and it can be seen that the bio-cellulose fibers and the nonwoven fabric fibers are not entangled. The lower image of FIG. 2 is a SEM image of the cross section of the bio-cellulose sheet according to one embodiment of the present disclosure, showing that the bio-cellulose fibers having nano-sized diameters and the microfibers of the nonwoven fabric exist in an entangled structure on one surface of the sheet.

### [BEST MODE FOR CARRYING OUT THE INVENTION]

According to an aspect of the present disclosure, there is provided a bio-cellulose sheet including a nonwoven fabric and bio-cellulose and having a structure in which the nonwoven fabric fiber and the bio-cellulose fiber are entangled.

In general, cellulose is a basic structural component of a cell wall of plant body, and refers to a cellulose constituting the plant fiber. For example, about 98% of cotton wool is composed of cellulose, and flax or hemp contain about 70% of cellulose. In the meantime, bio-cellulose is a high molecular weight polymer produced by culturing microorganisms, which is also referred to as microbial cellulose or bacterial cellulose.

It is known that the bio-cellulose-producing strains include *Acetobacter* sp., *Gluconacetobacter* sp., *Agrobacterium* sp., *Lactobacillus* sp., *Rhizobium* sp., *Pseudomonas* sp., and *Sarcina* sp., and the like. When these strains are cultured in a medium containing carbon and nitrogen, bio-cellulose in the form of a translucent pellicle is produced at the interface of the culture solution. The bio-cellulose thus formed has a high mechanical strength and a three-dimensional network structure composed of extremely fine pure cellulose.

In addition, bio-cellulose corresponds to a polymer bonded by β-1,4 bonds like plant-derived cellulose, and unlike plant-derived cellulose, it is produced in a pure state which does not include any impurities such as lignin, hemicelluloses, or pectin, and thus, separation and purification are easy. The bio-cellulose film of the network structure is a randomly formed fibrillated cellulose in the shape of a ribbon, which is composed of countless fine microfibrils having a width of smaller than 100 nm and a radius of 2 to 4 nm.

As such, the bio-cellulose has a fiber width much smaller than that of the plant-derived cellulose, and is composed of a three-dimensional network structure, and thus, is excellent in moisture-holding capacity and has a soft tough feeling and a smooth wearing feeling, like as a hydrogel mask. The bio-cellulose is suitable for use in cosmetics and medical fields because of its nano-structural properties, moisture-holding capacity, excellent wearing feeling, biocompatibility, and the like.

Bio-cellulose sheet refers to a sheet prepared in the shape that can be attached to the body by cutting bio-cellulose to a suitable thickness and/or shape. In this case, the sheet can be attached to the body by carrying cosmetic or medicinal ingredients in the form of a liquid, cream or emulsion. Unless otherwise defined hereon, the bio-cellulose sheet only refers to the sheet itself which does not include a support film releasably attached or laminated thereto.

The bio-cellulose sheet provided herein has a feature in that it includes a nonwoven fabric and bio-cellulose and having a structure in which the nonwoven fabric fiber and the bio-cellulose fiber are entangled. Therefore, from the viewpoint of having a structure in which the nonwoven fabric is included in the configuration of the bio-cellulose sheet itself, and it has a structure in which the nonwoven fibers and the bio-cellulose fibers are entangled, thus being unable to be detached from each other, the bio-cellulose sheet is distinguished from a nonwoven film which is further releasably attached or laminated to a conventional bio-cellulose sheet,

As used herein, the terms "entanglement" or "entangled structure" refer to a state in which the nonwoven fibers and the bio-cellulose fibers are entangled with each other like noodles. In this state, it is difficult to separate or detach the two fibers from each other, and accordingly, it is possible to integrally configure one sheet, without detaching the nonwoven fabric and the bio-cellulose from each other. In addition, such entanglement may impart high elasticity effects such as normal stress or elongation viscosity, or the tensile strength may be increased, thus allowing the sheet to become resistant to damage and providing handling properties that the handling is easy.

Nonwoven fabric is a fiber structure of a planar structure which is prepared by forming sheet-shaped webs that allow various fibers such as natural fiber, chemical fiber, glass fiber, metal fiber or the like to entangle according to their mutual properties, and bonding the webs by a mechanical or physical method. As a raw material fiber, one or more selected from the group consisting of natural fibers and synthetic fibers may be used. For example, it may be a nonwoven fabric material containing one or more selected from viscose rayon fiber, polypropylene fiber, polyethylene fiber, polyethylene terephthalate fiber, polyester fiber, nylon fiber and cellulose fiber. In the present disclosure, the material or preparation method of the nonwoven fabric is not particularly limited. The nonwoven fabric can be applied as long as it is those commonly used in the art. Preferably, the nonwoven fabric may be one or more types selected from chemical bonded nonwoven fabric, thermal bonded nonwoven fabric, Air Ray nonwoven fabric, Wet Ray nonwoven fabric, needle punched nonwoven fabric, spunlace nonwoven fabric (water-jet entanglement), spun bonded nonwoven fabric, melt blown nonwoven fabric, stitch bonded nonwoven fabric and electrospun nonwoven fabric.

The nonwoven fabric may be a commonly known spunlace-type nonwoven fabric, but is not limited thereto. The spunlace method is a method of bonding webs by cutting short fibers to stack the webs, followed by spraying high-pressure water flow, and it can obtain various designs according to the patterns of the plate installed on the lower part of the webs that receive high-pressure water flow, can be produced relatively thinly, has excellent flexibility and air permeability, is hygienic, and thus, is suitable as a material for beauty packs or medical patches.

The bio-cellulose sheet provided herein may be a sheet wherein bio-cellulose fibers are formed on one surface of the nonwoven fabric, which has a structure where the nonwoven fibers and the bio-cellulose fibers are entangled, and wherein the bio-cellulose fibers are not formed on the opposite side of the nonwoven fabric. The one surface of the nonwoven fabric on which the bio-cellulose fibers are formed has a structure in which the nonwoven fibers and the bio-cellulose fibers are entangled, and may be attached to the skin of the user's body. The one surface of the nonwoven fabric on which bio-cellulose is formed may include a plurality of protrusions so as to increase the adhesiveness to the skin and to allow the effective ingredients to penetrate deep into the skin through the capillaries of the skin, if necessary, but its shape is not particularly limited.

The nonwoven fibers may be microfibers. The nonwoven fabrics are composed of fibers having tens of micro-sized diameters, and bio-cellulose of several nano-sized diameters can be formed thereon.

In the present disclosure, the weight ratio of the nonwoven fibers and the bio-cellulose fibers may have various weight ratios depending on factors such as the type, shape, thickness, or density of the nonwoven fibers used even if the weight of the bio-cellulose weight is the same. Therefore, it will be apparent to those skilled in the art that the bio-cellulose sheet can be prepared by appropriately adjusting the weight ratio of the nonwoven fibers to the bio-cellulose fibers in consideration of various factors such as tears, handling, adhesive strength, wearing feeling, absorbency, or productivity (e.g., production time, cost, production amount), while sufficiently forming bio-cellulose on the nonwoven fabric.

In another embodiment, the present disclosure provides a bio-cellulose sheet having an entanglement strength of greater than 0 N/m, particularly 0.1 N/m or higher, 0.2 N/m or higher, 0.3 N/m or higher, 0.4 N/m or higher, 0.5 N/m or higher, 0.6 N/m or higher, 0.7 N/m or higher, 0.8 N/m or higher, 0.9 N/m or higher, or 1.0 N/m or higher. In addition, even if the entanglement strength is measured to be high, there may be no big problem in terms of the physical properties. However, those skilled in the art can adjust the entanglement strength to a desired range in consideration of various factors such as tears, handling, adhesive strength, wearing feeling, absorbency, or productivity (e.g., production time, cost, production amount), even while bio-cellulose being sufficiently formed on the nonwoven fibers. For example, it may be adjusted to 100 N/m or less, 90 N/m or less, 80 N/m or less, 70 N/m or less, 60 N/m or less, 50 N/m or less, 40 N/m or less, 30 N/m Or less, 20 N/m or less, or 10 N/m or less, but the present disclosure is not limited thereto.

According to one embodiment of the present disclosure, it was confirmed that the fiber entanglement strength of the nonwoven fabric film releasably attached to the bio-cellulose sheet is almost 0, whereas the entanglement strength of the bio-cellulose and the nonwoven fibers included in the sheet of the present disclosure exhibits a value significantly higher than that of the nonwoven fabric film, thereby producing a sheet having a structure in which the nonwoven fibers and the bio-cellulose fibers are entangled. Furthermore, by appropriately changing specific culture conditions (e.g., culturing period, concentration of inoculated bacteria, amount of culture solution, and the like) during the production of the bio-cellulose sheet, it was confirmed that the mechanical strength of the bio-cellulose sheet can be appropriately adjusted, or the preparation amount of bio-cellulose can be easily adjusted as necessary, even while maintaining the structure in which the nonwoven fibers and the bio-cellulose fibers are entangled.

In still another embodiment, the present disclosure provides a bio-cellulose sheet having a tensile strength of greater than 0 MPa, specifically 0.1 MPa or higher, 0.2 MPa or higher, 0.3 MPa or higher, 0.4 MPa or higher, 0.5 MPa or higher, 0.6 MPa or higher, 0.7 MPa or higher, 0.8 MPa or higher, 0.9 MPa or higher, or 1.0 MPa or higher. In addition, even if the tensile strength is measured to be high, there may be no big problem in terms of the physical properties. However, those skilled in the art can adjust the tensile strength within a desired range in consideration of various factors such as tears, handling, adhesive strength, wearing feeling, absorbency, or productivity (e.g., production time, cost, production amount), even while bio-cellulose being sufficiently formed on the nonwoven fibers. For example, it may be adjusted to 100 MPa or less, 90 MPa or less, 80 MPa or less, 70 MPa or less, 60 MPa or less, 50 MPa or less, 40 MPa or less, 30 MPa or less, 20 MPa or less, or 10 MPa or less, but the present disclosure is not limited thereto.

According to one embodiment of the present disclosure, it was confirmed that the fiber tensile strength of the nonwoven fabric film releasably attached to the bio-cellulose sheet is almost 0, whereas the tensile strength of the sheet of the present disclosure exhibits a value significantly higher than that of the nonwoven fabric film, thereby producing a sheet having a structure in which the nonwoven fibers and the bio-cellulose fibers are entangled. Furthermore, by appropriately changing specific culture conditions (e.g., culturing period, concentration of inoculated bacteria, amount of culture solution, and the like.) during the production of the bio-cellulose sheet, it was confirmed that the mechanical strength of the bio-cellulose sheet can be appropriately adjusted, or the production amount of bio-cellulose can be easily adjusted as necessary, even while maintaining the structure in which the nonwoven fibers and the bio-cellulose fibers are entangled.

According to another aspect of the present disclosure, there is provided a method for producing a bio-cellulose sheet including the steps of: impregnating the nonwoven fabric with a bio-cellulose culture solution; and culturing the nonwoven fabric impregnated with the culture solution.

The bio-cellulose culture solution contains nutrients necessary for the growth and development of bio-cellulose-producing microorganisms. A conventional culture solution used in the art to produce bio-cellulose may be used. The culture solution may contain various carbon sources, nitrogen sources and/or components of trace elements. For example, a culture solution containing alcohols, proteins, and yeasts may be used.

Specifically, as the carbon sources, carbohydrates such as glucose, lactose, sucrose, fructose, maltose, starch and cellulose; oils and fats such as soybean oil, sunflower oil, castor oil and coconut oil; fatty acids such as palmitic acid, stearic acid and linoleic acid; alcohols such as glycerol and ethanol; and organic acids such as acetic acid may be included. These carbon sources may be used alone or in combination.

As the nitrogen sources, organic nitrogen sources such as peptone, yeast extract, gravy, malt extract, corn steep liquor (CSL), soybean flour; and inorganic nitrogen sources such as urea, ammonium sulfate, ammonium chloride, ammonium phosphate, ammonium carbonate, and ammonium nitrate may be included. These nitrogen sources may be used alone or in combination.

If necessary, the culture solution may additionally include phosphorus sources such as potassium dihydrogen phosphate, dipotassium hydrogen phosphate, and corresponding sodium-containing salts. In addition, metals such as magnesium sulfate or iron sulfate may be included. Further, amino acids, vitamins, and suitable precursors may be added.

Further, the culture solution may include a plant extract containing glucose so as to increase the production efficiency of bio-cellulose. For example, it may include plant materials such as coconut, sugarcane, Korean raspberry (bokbunja), tangerine, pear, apple, grape, watermelon, and corn. In addition, any culture solution capable of producing bio-cellulose by fermenting strains can be used without particular limitation.

The bio-cellulose sheet may be produced by impregnating a nonwoven fabric into a bio-cellulose culture solution inoculated with a strain and then removing the fabric, or culturing the nonwoven fabric in an impregnated state to produce bio-cellulose on the nonwoven fabric. As a strain inoculated into the culture solution and as a bio-cellulose-synthesizing strain, a microbial strain belonging to *Acetobacter* sp., *Gluconacetobacter* sp., *Agrobacterium* sp., *Lactobacillus* sp., *Rhizobium* sp., *Pseudomonas* sp., and *Sarcina* sp., and the like may be used, but is not limited thereto. For example, *Acetobacter xylinum, Acetobacter xylinus, Acetobacter pasteurinanus, A. hansenii, Gluconacetobacter xylinus,* or the like may be used. A single strain may be cultured, or multiple strains may be mixed and cultured to obtain a higher production efficiency. For example, a mixture of *Acetobacter xylinum* having an excellent production yield and bacteriocin-producing lactic acid bacteria for preventing prevent bacterial contamination may be subjected to static cultivation or shake cultivation.

When the strain is cultured, the pH is suitably in the range of 3 to 7, but is not limited thereto, and can be suitably adjusted depending on the inoculated bacteria. In addition, the culture temperature may be in the range 20°C to 40°C, more particularly 20°C to 30°C, and the culture time may be 1 day to 10 days, but the specific culture temperature or time may be appropriately adjusted to maintain the optimum growth conditions of bio-cellulose-producing microorganisms. Through such culture, the microorganisms break down organic matters using their own enzymes to produce bio-cellulose, and this process is referred to as fermentation.

In addition, it will be understood by those skilled in the art that the amount of medium to be added and the culture time may vary depending on the thickness of the bio-cellulose to be produced. If necessary, an additional medium may be added to prevent the nonwoven fabric from drying out during the culture period.

As methods for culturing strains, any culture method commonly used in the art to which the present disclosure pertains, such as a static cultivation method or an agitated cultivation method, may also be used, but the static cultivation method may be commonly used.

The static cultivation method is a method in which bacteria are first transplanted into a medium, and then cultured in a flask while the flak is placed on a shelf for hours to days. For example, *Acetobacter xylinum* is an aerobic bacterium that produces a thin, elastic film on the surface of the medium. The formed cellulose biofilm can be obtained as pure cellulose simultaneously by removing excess bacteria with an aqueous sodium hydroxide solution. Thereafter, bio-cellulose can be obtained by repeatedly washing with distilled water.

The shaking culture method, which is another method, is a method of culturing while continuously stirring at a constant rate in a shaking incubator using a liquid medium. The thus-formed bio-cellulose may become smaller in size compared to the degree of crystallinity of the bio-cellulose cultured by static cultivation.

The bio-cellulose sheet may be immediately obtained in a desired shape by impregnating a nonwoven fabric cut into a shape suitable for a body part to be attached, or alternatively, after the sheet is first prepared, a step of cutting a nonwoven fabric into a shape suitable for the body to be attached thereto is carried out to obtain a desired shape.

In order for the bio-cellulose to be used as a beauty pack sheet, it is necessary to kill the microbial cells injected for fermentation and remove the culture solution. Although not limited thereto, a hot water sterilization process may be repeatedly performed to kill the microbial cells and remove the culture solution.

The prepared bio-cellulose sheet can be provided by slicing at an appropriate thickness. Although the thickness range is not particularly limited, when the thickness of bio-cellulose gets thinner, the adhesive strength to the skin may increase, but the amount of liquid retained in the sheet may decrease and the sheet may be quickly dried on the skin. Further, when the thickness gets thicker, the moisturizing power may be maintained on the skin for a long time, but the adhesive strength to the skin may be decreased, thus reducing convenience of use. Therefore, the sheet may usually be provided with a thickness of 0.1 mm to 5.0 mm, a thickness of 0.5 mm to 3.5 mm, or a thickness of 0.05 mm to 1 mm. When it is used as a beauty sheet by injecting cosmetic ingredients or pharmaceutical ingredients, the above range may be suitable for providing a high moisture content while being easily attached to the body.

As for a conventional bio-cellulose sheet, after a bio-cellulose sheet is prepared from a bio-cellulose culture solution inoculated with microorganisms, a supporter such as a nonwoven fabric film, a reticulated fiber cloth film, a polymer thin film, a waterproof cloth film, a polymer-foam film, or a paper-like film is laminated on one or both sides of the prepared bio-cellulose sheet to maintain the appearance of the sheet, and the user has to remove the film immediately before use.

In contrast, the bio-cellulose sheet provided herein is prepared by impregnating a nonwoven fabric with a bio-cellulose culture solution, so that bio-cellulose is formed on the non-woven fabric, and thus is formed in a structure in which the nonwoven fabric fibers and the bio-cellulose fibers are entangled with each other. Accordingly, the bio-cellulose sheet of the present disclosure is different from the conventionally commercialized bio-cellulose sheet to which the non-woven fabric is attached, from the perspective that the nonwoven fabric and bio-cellulose are not detachable from each other and integrally constitute the sheet.

The produced bio-cellulose sheet may be dehydrated or dried to make into a constant moisture-holding state. When a cosmetic emulsion or the like is deposited after complete drying, the water-holding capacity of bio-cellulose may be decreased, which may increase the time for swelling of the sheet, or the sheet may not be sufficiently swollen. Thus, the moisture content may be maintained in a weight of 1 to 50 times the dry weight of the bio-cellulose sheet through a dehydration process, and then a cosmetic emulsion or the like may be introduced therein by impregnating with an impregnation liquid. More specifically, the moisture content may be maintained in a weight range of 10 to 20 times the dry weight of the sheet.

The prepared bio-cellulose sheet may be used as a beauty pack by injecting a cosmetic ingredient having a special desired purpose such as whitening, anti-wrinkle, and supplying of nutrition, or the like, or may be used as a medical patch for treatment of burns or wounds. For example, it may be used as a mask pack that covers the entire face, or a beauty pack applied to specific areas such as under the eyes, eyes, nose, mouth, cheeks, neck, or chin, an anti-inflammatory patch for patients with bruises, a patch for patients with arthritis for long-term use, an itching-calming coolant for patients with atopic dermatitis, a wound-protecting patch and botox patch that can be used for cosmetic surgery, or the like but is not limited thereto.

According to still another aspect of the present disclosure, there is provided a beauty pack including the bio-cellulose sheet. In addition, there is provided a cosmetic pack containing cosmetic ingredients in the sheet. According to still further another aspect of the present disclosure, there is provided a medical patch including the bio-cellulose sheet.

The cosmetic ingredient may include various functional ingredients depending on the purposes, such as whitening, anti-wrinkle, improvement of skin elasticity, enhancement of skin immunity, moisturization, supplying of nutrition, blemish removal, alleviation of skin problems, skin anti-inflammation, and antioxidation. For example, it may include one or more selected from glycerin, probiotic extract, trehalose, glucan, chitosan, vitamins, ceramide, collagen, hyaluronic acid, nicotinamide, adenosine, retinol, allantoin, arbutin, retinol, antioxidants, aroma oil, epidermal growth factor, platelet-derived growth factor, insulin growth factor, tea tree, polyphenols, anthocyanidin, isoflavone, lutein, carotene, carotenoid, natural extracts (aloe extract, lily extract, seaweed extract, rose extract, persimmon extract, jasmine extract, Buddha's hand extract, mushroom extract, bamboo extract, apricot seed extract, cucumber extract, green tea extract, ginseng extract, red ginseng extract, mung bean extract, mugwort extract, or the like), or a mixture or two or more thereof, but is not limited thereto. These effective ingredients may be impregnated into the sheet in the form of an emulsion, more specifically an oil-in-water type emulsion.

### [MODE FOR CARRYING OUT THE INVENTION]

Hereinafter, the present disclosure will be described by way of Example. However, these Examples are given for illustrative purposes only, and the scope of the present disclosure is not limited by these examples.

### Example 1: Preparation of Bio-cellulose Sheet

As Example, a culture solution containing 25 g of mannitol, 5 g of peptone, and 3 g of yeast was used as the bio-cellulose culture solution, based on 1 L of the culture solution, and 5 ml of *Gluconacetobacter xylinus* (obtained from Korean Culture Center of Microorganisms) was inoculated into the culture solution. Specifically, 1/10 of G. *xylinus* cultured in a 90 mm Petri dish containing a solid medium collected and released into 50 ml of the culture solution having the composition described above. A miracloth nonwoven fabric (Sigma-Aldrich) was immersed into the culture solution added with the bacteria, and then taken out and spread on an empty Petri dish. A common medium, to which no bacteria was added, was added thereto so that the nonwoven fabric was submerged. Thereafter, the Petri dish was covered with a lid and sealed, and then subjected to static cultivation for more than 3 days in a 26°C incubator.

As Comparative Example, the bio-cellulose culture solution having the same composition as above was subjected to static cultivation to produce a bio-cellulose sheet.

As a result of observation with a scanning electron microscope (SEM), in the case of the bio-cellulose sheet of Example, bio-cellulose was not formed on one side of the sheet and only the nonwoven fibers were observed, and the bio-cellulose having nano-sized diameters entangled with the micro-sized fibers of the nonwoven fabric was observed on the other side of the sheet (FIG. 1).

### Example 2: Measurement of Entanglement Strength of Bio-cellulose Sheet

The 180° peel test was performed with the UTM machine (Universal testing machine LRX plus model from Lloyd Instruments), and the entanglement strength of the bio-cellulose sheet of Example and the bio-cellulose sheet of Comparative Example, which were prepared in Example 1, was measured. The measurement conditions and results are as follows:
Specimen Specification: 25*100mm
Load cell: 10N
Propagation speed: 25mm/min

As a result of measuring the entanglement strength by Peel test through repeated experiments, the entanglement strength of the bio-cellulose sheet of Example was found to be 27.30±2.23 N/m, and the entanglement strength of the bio-cellulose sheet of Comparative example was found to be 0.30±0.13 N/m, showing that the sheet of Example had about 90 times stronger entanglement strength.

Actually, in the case of Comparative Example, the entanglement strength should be 0 since no entanglement was observed, but some entanglement strength was measured due to the tension of the cosmetic water.

Next, the bio-cellulose sheet was prepared in the same manner as in Example 1, except that the culture period was reduce to 2 days, and the entanglement strength thereof was measured by the peel test under the same measurement conditions as described above. As a result, the entanglement strength was found to be 18.45±3.81 N/m, showing that it had about 60 times stronger entanglement strength compared to the entanglement strength of Comparative Example, which was 0.30±0.13 N/m.

In addition, the bio-cellulose sheet was prepared in the same manner as in Example 1, except that the culture period was increased to 10 days, and the entanglement strength thereof was measured under the same measurement conditions as described above. As a result, the entanglement strength was found to be 33.18±5.42 N/m, showing that it had about 110 times stronger entanglement strength compared to the entanglement strength of Comparative Example.

As such, it can be confirmed that the sheet with the structure in which the nonwoven fibers and the bio-cellulose fibers are entangled could be prepared by the method of the present disclosure. Furthermore, it can be confirmed that even when the method for preparing a bio-cellulose sheet was appropriately changed (e.g., culture period), the bio-cellulose sheet having an excellent entanglement strength could still be prepared. Therefore, those skilled in the art can easily adjust the mechanical strength or the preparation amount of bio-cellulose by appropriately changing the specific culture conditions at the time of preparing the bio-cellulose sheet, in consideration of the adhesive strength, wearing feeling, or absorbency of the bio-cellulose sheet, while maintaining the structure in which the nonwoven fibers and the bio-cellulose fibers are entangled.

### Example 3: Measurement of Tensile Strength of Bio-cellulose Sheet

In order to support the fact that the bio-cellulose sheet according to the present disclosure is resistant to damage and can be easily handled, the tensile strength of the bio-cellulose sheet of Example and the bio-cellulose sheet of Comparative Example, which were prepared in Example 1, was measured. The tensile strength was performed using the UTM machine (5982 model from INSTRON) under the following conditions:
Specimen Specification: 25*100mm
Load cell: 5KN
Propagation speed: 15mm/min

As a result of measuring the tensile strength, the tensile strength of the bio-cellulose sheet of Example was found to be 2.75±0.44 MPa, and the tensile strength of the bio-cellulose sheet of Comparative Example was found to be 0.085±0.005 MPa, showing that the sheet of Example had about 32 times stronger tensile strength.

Next, the bio-cellulose sheet prepared in the same manner as in Example 1, except that the culture period was reduce to 2 days, and the tensile strength thereof was measured under the same measurement conditions as described above. As a result, the tensile strength was found to be 1.23±0.28 N/m, showing that it had about 14 times stronger tensile strength compared to the tensile strength of Comparative Example.

In addition, the bio-cellulose sheet was prepared in the same manner as in Example 1, except that the culture period was increased to 10 days, and the tensile strength was measured under the same measurement conditions as described above. As a result, the tensile strength was found to be 7.48±0.57 N/m, showing that it had about 88 times stronger tensile strength compared to the tensile strength of Comparative Example.

As such, it can be confirmed that the sheet with the structure in which the nonwoven fibers and the bio-cellulose fibers are entangled could be prepared by the method of the present disclosure. Furthermore, it can be confirmed that even when the method for preparing a bio-cellulose sheet was appropriately changed (e.g., culture period), the bio-cellulose sheet having an excellent tensile strength could still be prepared. Therefore, those skilled in the art can easily adjust the mechanical strength or the preparation amount of bio-cellulose by appropriately changing the specific culture conditions at the time of preparing the bio-cellulose sheet, in consideration of the adhesive strength, wearing feeling, or absorbency of the bio-cellulose sheet, while maintaining the structure in which the nonwoven fibers and the bio-cellulose fibers are entangled.

While the present disclosure has been described with reference to the particular illustrative embodiments, it will be understood by those skilled in the art that the present disclosure may be embodied in other specific forms without departing from the technical spirit or essential characteristics of the present disclosure. Therefore, the embodiments described above are considered to be illustrative in all respects and not restrictive . Furthermore, the scope of the present disclosure is defined by the appended claims rather than the detailed description, and it should be understood that all modifications or variations derived from the meanings and scope of the present disclosure and equivalents thereof are included in the scope of the appended claims.

## Claims

1. A bio-cellulose sheet comprising a nonwoven fibers and bio-cellulose, and having a structure in which the nonwoven fibers and the bio-cellulose fibers are entangled.

2. The bio-cellulose sheet of claim 1, wherein the structure in which the nonwoven fibers and the bio-cellulose fibers are entangled is formed on one surface of the nonwoven fibers, and the bio-cellulose fibers is not formed on the opposite surface of the nonwoven fibers.

3. The bio-cellulose sheet of claim 1 or 2, wherein the sheet has a tensile strength of 1.0 MPa or more.

4. The bio-cellulose sheet of any one of claims 1 to 3, wherein the sheet has an entanglement strength of 1.0 N/m or more.

5. The bio-cellulose sheet of any one of claims 1 to 4, wherein the nonwoven fibers includes microfibers.

6. The bio-cellulose sheet of any one of claims 1 to 5, wherein the nonwoven fibers is one or more selected from the group consisting of natural fibers and synthetic fibers.

7. A method for preparing the bio-cellulose sheet of any one of claims 1 to 6, comprising the steps of:
impregnating a nonwoven fiber with a bio-cellulose culture solution; and
culturing the nonwoven fiber impregnated with the culture solution.

8. The method of claim 7, wherein the culture solution comprises alcohols, proteins, yeasts, and microorganisms.

9. The method of claim 7, wherein the culturing is carried out by static cultivation.

10. A beauty pack comprising the bio-cellulose sheet of any one of claims 1 to 6.

11. The beauty pack of claim 10, wherein cosmetic ingredients are contained in the sheet.

12. A medical patch comprising the bio-cellulose sheet of any one of claims 1 to 6.
